# EUROPÄISCHE PATENTSCHRIFT

(11) **EP 2 706 247 B1**
(45) Veröffentlichungstag und Bekanntmachung des Hinweises auf die Patenterteilung: **12.07.2017**
(21) Anmeldenummer: 13195253.3
(22) Anmeldetag: 30.04.2012
(51) Int. Cl.: F16C 19/46, F16C 33/46, A61B 17/16, F16C 19/49

(54) **Chirurgisches Instrument, chirurgisches Handstück und chirurgisches Antriebssystem**
Surgical instrument, surgical hand-held tool and surgical drive system
Instrument chirurgical, pièce à main chirurgicale et système d'entraînement chirurgical

(30) Priorität: 06.05.2011 DE 102011050193
(43) Veröffentlichungstag der Anmeldung: 12.03.2014
(62) Teilanmeldung aus: 12166169.8
(73) Patentinhaber: AESCULAP AG, 78532 Tuttlingen (DE)
(72) Erfinder: Blust, Edgar, 78126 Königsfeld (DE)
(74) Vertreter: Hoeger, Stellrecht & Partner Patentanwälte mbB

(56) Entgegenhaltungen:
- WO-A1-2005/119074
- CH-A- 296 826
- DE-A1-102005 010 881
- DE-A1-102006 044 802
- DE-B- 1 055 752
- DE-B- 1 189 229
- DE-B- 1 215 446
- DE-U1-202011 050 062
- JP-A- S59 155 622

## Beschreibung

Die vorliegende Erfindung betrifft ein chirurgisches Instrument umfassend einen Schaft und eine im Schaft rotierbar gelagerte Antriebswelle, welche an ihrem distalen Ende ein Werkzeugelement trägt oder umfasst, wobei im distalen Endbereich des Schafts ein Radiallager angeordnet oder ausgebildet ist zum rotierbaren Lagern der Antriebswelle am Schaft, wobei das Radiallager in Form eines Nadellagers ausgebildet ist, wobei das Nadellager einen Lagerkäfig umfasst, in welchem eine Mehrzahl von Lagernadeln parallel zu einer Längsachse des Nadellagers rotierbar gelagert sind.

Ferner betrifft die vorliegende Erfindung ein chirurgisches Handstück umfassend einen in einem Gehäuse angeordneten Antrieb.

Und schließlich betrifft die Erfindung ein chirurgisches Antriebssystem umfassend mindestens ein chirurgisches Handstück mit einem in einem Gehäuse angeordneten Antrieb und eine Steuer- und/oder Regelungsvorrichtung zum Steuern und/oder Regeln des Antriebs.

Chirurgische Instrumente der eingangs beschriebenen Art werden insbesondere in der Chirurgie eingesetzt. Sie werden mit Antrieben umfassenden Handstücken gekoppelt, um die Antriebswelle in Rotation zu versetzen. Ein Problem bei derartigen Instrumenten ist die Lagerung der Antriebswelle. Dies liegt zum einen an einer zunehmenden Miniaturisierung der Systeme, welche zu einer Reduzierung eines Außendurchmessers der Schäfte der Instrumente führt. Es ist bekannt, zur Lagerung der Schäfte Radiallager in Form von Kugellagern zu verwenden. Allerdings können diese nicht beliebig klein ausgebildet werden. Zum anderen ist zu berücksichtigen, dass derartige Instrumente mit sehr hohen Drehzahlen bis zu 100.000 Umdrehungen pro Minute betrieben werden. Damit verbunden sind Probleme im Zusammenhang mit Abrieb und erhöhter Erwärmung.

Chirurgische Instrumente der eingangs beschriebenen Art sind beispielsweise aus der DE 1 189 229 B bekannt, dass die Merkmale des Oberbegriffs von Anspruch 1 offenbart. In der JP S59 155622 A ist ein Haltering für ein Rollenlager beschrieben.

Es ist daher eine Aufgabe der vorliegenden Erfindung, ein chirurgisches Instrument, ein chirurgisches Handstück sowie ein chirurgisches Antriebssystem der eingangs beschriebenen Art so zu verbessern, dass eine Montage derselben vereinfacht wird.

Diese Aufgabe wird bei einem chirurgischen Instrument der eingangs beschriebenen Art erfindungsgemäß dadurch gelöst, dass der Lagerkäfig zwei miteinander verbundene Lagerkäfigteile umfasst, dass das Instrument eine Verbindungseinrichtung zum Verbinden der beiden Lagerkäfigteile umfasst und dass das Instrument eine Nadellagersicherungseinrichtung zum axialen Sichern des Nadellagers am Schaft umfasst.

Nadellager haben im Vergleich zu Kugellagern eine deutlich höhere Tragzahl und können somit deutlich höhere Kräfte aufnehmen, ohne dabei Schaden zu nehmen. Das Nadellager bildet vorzugsweise das distalste Radiallager am Schaft, welches keine axialen Kräfte aufnehmen muss. Nadellager können keine axialen Kräfte aufnehmen, so dass sie an dieser Stelle des Schafts Nadellager als Radiallager hervorragend geeignet sind. Insgesamt können Nadellager bei gleicher Baugröße, also gleichem Außendurchmesser, deutlich höhere Kräfte aufnehmen als Kugellager. Mit anderen Worten können die gleichen Kräfte von Nadellagern aufgenommen werden, die im Vergleich zu Kugellagern einen deutlich kleineren Außendurchmesser aufweisen. Um insbesondere eine Montage des Instruments zu vereinfachen, ist es vorteilhaft, dass das Nadellager einen Lagerkäfig umfasst, in welchem eine Mehrzahl von Lagernadeln parallel zu einer Längsachse des Nadellagers rotierbar gelagert ist. Vorzugsweise kann das Nadellager derart ausgebildet sein, dass es keinen inneren, vorzugsweise drehfest und axial an der Antriebswelle gesicherten Lagerring aufweist, sondern dass die Lagernadeln direkt an der Antriebswelle anliegen und sich an dieser abwälzen können. Auf diese Weise können eine Baugröße des Nadellagers und damit insbesondere auch ein Außendurchmesser des Schafts weiter reduziert werden. Die Montage des Instruments lässt sich weiter insbesondere dadurch vereinfachen, dass der Lagerkäfig zwei miteinander verbundene Lagerkäfigteile umfasst. Beispielsweise können so die Lagernadeln und auch entsprechende Sicherungselemente, beispielsweise Kugeln, auf einfache Weise am Lagerkäfig montiert werden. Beispielsweise können sie in einen der beiden Lagerkäfigteile eingesetzt und dann mit dem zweiten Lagerkäfigteil beim Verbinden desselben mit dem ersten Lagerkäfigteil unverlierbar am Lagerkäfig gesichert werden. Ferner umfasst das Instrument eine Verbindungseinrichtung zum Verbinden der beiden Lagerkäfigteile. Die Verbindungseinrichtung ermöglicht es insbesondere, die Lagerkäfigteile unverlierbar miteinander zu verbinden. Außerdem umfasst das Instrument eine Nadellagersicherungseinrichtung zum axialen Sichern des Nadellagers am Schaft. So kann auf einfache Weise das Nadellager gegen eine axiale Bewegung relativ zum Schaft gesichert werden.

Günstigerweise umfasst das Nadellager mindestens drei Lagernadeln. Mit diesen kann insbesondere eine 3-Punktauflage für die Antriebswelle ausgebildet werden, und das bei kleinstmöglicher Baugröße.

Um eine Stabilität des Nadellagers zu erhöhen, ist es vorteilhaft, wenn mindestens zwei Lagernadeln eine Nadelgruppe definieren und wenn das Nadellager mindestens zwei Nadelgruppen umfasst. Beispielsweise kann so ein Nadellager mit mindestens vier Lagernadeln ausgebildet werden, die paarweise zu einer Nadelgruppe angeordnet sind. Denkbar sind beispielsweise auch zwei Nadelgruppen mit drei Lagernadeln oder drei Nadelgruppen mit jeweils zwei oder drei Lagernadeln.

Des Weiteren kann es vorteilhaft sein, wenn ein Abstand benachbarter Nadelgruppen voneinander in Umfangsrichtung größer ist als ein Abstand benachbarter Lagernadeln einer Nadelgruppe voneinander in Umfangsrichtung. Mit anderen Worten können so Nadelgruppen in Umfangsrichtung voneinander räumlich getrennt werden. Ein größerer Abstand ermöglicht es, beispielsweise weitere Elemente zwischen den Nadelgruppen am Lagerkäfig vorzusehen, die zum Beispiel den Lagerkäfig axial am Schaft sichern.

Günstigerweise ist das Nadellager axial gesichert am Schaft gehalten ist. Auf diese Weise kann verhindert werden, dass es sich in einer Richtung parallel zu einer Längsachse der Antriebswelle relativ zum Schaft bewegen kann.

Besonders günstig ist es, wenn die Nadellagersicherungseinrichtung mindestens ein erstes Sicherungselement und mindestens ein zweites Sicherungselement umfasst, wenn das eine der mindestens einen ersten und zweiten Sicherungselemente am Nadellager angeordnet oder gehalten ist, wenn das andere der mindestens einen ersten und zweiten Sicherungselemente am Schaft angeordnet oder gehalten ist und wenn das mindestens eine erste Sicherungselement und das mindestens eine zweite Sicherungselement in einer Sicherungsstellung miteinander in Eingriff stehen. Auf diese Weise kann die Nadellagersicherungseinrichtung besonders einfach und kompakt ausgebildet werden. Insbesondere lassen sich so auch Montagebedürfnisse konstruktiv individuell berücksichtigen, um den Zusammenbau des Instruments zu vereinfachen.

Günstig ist es, wenn das mindestens eine erste Sicherungselement in Form eines Sicherungsvorsprungs ausgebildet ist, wenn das mindestens eine zweite Sicherungselement in Form einer Sicherungsausnehmung ausgebildet ist und wenn der Sicherungsvorsprung und die Sicherungsausnehmung in der Sicherungsstellung miteinander in Eingriff stehen. Insbesondere können sowohl der Sicherungsvorsprung als auch die Sicherungsausnehmung in einer Richtung quer zu einer vom Nadellager definierten Längsachse orientiert sein, um so optimal eine axiale Sicherung des Nadellagers am Schaft zu erreichen.

Vorteilhaft ist es, wenn der Lagerkäfig eine Längsachse definiert und eine die Längsachse konzentrisch umgebende Innenfläche und eine die Längsachse konzentrisch umgebende Außenfläche umfasst und wenn das mindestens eine erste Sicherungselement über die Außenfläche vorragt beziehungsweise über diese vorsteht, nicht jedoch über die Innenfläche. Auf diese Weise kann verhindert werden, dass das mindestens eine erste Sicherungselement mit der Antriebswelle in Berührung kommt. Damit kann sichergestellt werden, dass das mindestens eine erste Sicherungselement ausschließlich zur axialen Sicherung des Nadellagers am Schaft dient und nicht durch Reibung an der Antriebswelle Überhitzen kann. Außerdem wird so auf einfache Weise ein Wechsel der Antriebswelle ermöglicht. Dies ist insbesondere wünschenswert, wenn das Werkzeugelement unlösbar mit der Antriebswelle verbunden, insbesondere einstückig mit dieser ausgebildet ist. Das mindestens eine erste Sicherungselement kann beispielsweise in Form einer einen Sicherungsvorsprung definierenden Kugel ausgebildet sein, welche in eine Ringnut des Schafts oder einer an diesem gehaltenen Hülse beziehungsweise einem an diesem gehaltenen Lagerring eintaucht und so den Lagerkäfig axial am Schaft sichert.

Vorzugsweise sind der Sicherungsvorsprung in Form einer Kugel und die Sicherungsausnehmung in Form einer zur Kugel korrespondierenden Ringnut ausgebildet. Die Verwendung von Kugeln als Sicherungsvorsprünge hat insbesondere den Vorteil, dass hier bei sehr hohen Drehzahlen im Bereich von 80.000 Umdrehungen pro Minute bis 100.000 Umdrehungen pro Minute gegenüber einfachen seitlichen Anschlägen ein Abrieb sowie eine Erwärmung vermindert werden kann. Ferner kann so auch eine Montage des Lagerkäfigs am Schaft vereinfacht werden. Sowohl die Lagernadeln als auch die Kugeln sind vorzugsweise in entsprechenden Ausnehmungen am Lagerkäfig gehalten. Günstigerweise sind diese konisch geformt, um zu verhindern, dass weder die Lagernadeln noch die Kugeln in Richtung auf die Längsachse hin aus dem Lagerkäfig herausfallen können.

Besonders günstig ist es, wenn die Ringnut in Richtung auf eine Längsachse des Nadellagers hin geöffnet ist. So können die Kugeln als radiale Sicherungsvorsprünge vom Lagerkäfig ab- oder vorstehend in die Ringnut eingreifen und das Nadellager axial am Schaft sichern.

Grundsätzlich wäre es denkbar, die Sicherungsausnehmung direkt am Schaft auszubilden. Günstig ist es jedoch, wenn die Sicherungsausnehmung an einem Haltering ausgebildet ist, welcher axial und drehfest am Schaft festgelegt ist. Eine solche Ausgestaltung vereinfacht die Herstellung des Instruments, da an einem Haltering eine in einer Innenwand vorgesehene Ringnut deutlich einfacher herzustellen ist als an einem langgestreckten Schaft. Ferner kann der Haltering auch aus einem anderen Material hergestellt werden als der Schaft, wodurch sich gezielt eine Stabilität erhöhen und ein Abrieb entsprechend der Wahl der Materialien verringern lässt. Der Haltering kann insbesondere in den Schaft eingepresst, also mit Presssitz gehalten, oder zwischen zwei in axialer Richtung wirkenden Anschlägen am Schaft gehalten sein.

Vorteilhaft ist es, wenn zwischen zwei Lagernadeln ein Sicherungsvorsprung am Lagerkäfig angeordnet oder ausgebildet ist. So kann beispielsweise einerseits nach innen ein Radiallager durch die Lagernadeln ausgebildet werden für die Antriebswelle und andererseits nach außen das Nadellager am Schaft mittels des Sicherungsvorsprungs gesichert werden. Ist der Sicherungsvorsprung in Form einer Kugel ausgebildet, kann am Lagerkäfig eine entsprechende Ausnehmung oder Vertiefung vorgesehen sein. Dabei muss es sich nicht zwingend um eine Durchbrechung handeln, da vorzugsweise der Haltering derart ausgebildet ist, dass die Kugel nicht nach außen herausfallen kann. Beispielsweise können so drei Lagernadeln eine Dreipunktauflage für die Antriebswelle bilden und drei Sicherungsvorsprünge ein Dreipunktlager zum Halten des Lagerkäfigs am Schaft.

Günstig kann es ferner auch sein, wenn zwischen zwei Nadelgruppen ein Sicherungsvorsprung am Lagerkäfig angeordnet oder ausgebildet ist. Auf diese Weise kann die Zahl der durch die Lagernadeln definierten Anlagelinien an der Antriebswelle in gewünschter Weise erhöht werden, was eine weitere Erhöhung einer Tragzahl des Nadellagers zur Folge hat.

Um eine möglichst gute Sicherung des Nadellagers am Schaft zu gewährleisten, ist es vorteilhaft, wenn mindestens zwei Sicherungsvorsprünge vorgesehen sind. Diese sind dann idealerweise um 180° versetzt am Lagerkäfig angeordnet oder gehalten. Bei drei Sicherungsvorsprüngen bildet sich ebenfalls eine gleichmäßige Verteilung derselben über den Umfang des Lagerkäfigs an, so dass bei drei Sicherungsvorsprüngen eine 120°-Teilung günstig ist.

Vorteilhaft ist es, wenn das Instrument mindestens zwei in Längsrichtung des Nadellagers versetzt angeordnete erste und/oder zweite Sicherungselemente umfasst. Eine solche Anordnung kann eine axiale Sicherung des Nadellagers am Schaft verbessern. Beispielsweise können zwei Paare von jeweils zwei axial zueinander versetzten Sicherungselementen um 180° in Umfangsrichtung versetzt am Lagerkäfig angeordnet oder ausgebildet sein.

Um eine möglichst hohe Belastbarkeit des Nadellagers zu erreichen, ist es günstig, wenn die Zahl der Lagernadeln einem ganzzahligen Vielfachen der Zahl der ersten oder zweiten Sicherungselemente entspricht. Beispielsweise können zwei Sicherungselemente in Form von Kugeln und vier Lagernadeln, die jeweils eine zwei Lagernadeln umfassende Nadelgruppe bilden, vorgesehen sein. Denkbar sind auch, insgesamt sechs oder acht Lagernadeln vorzusehen, die in Gruppen von zwei oder drei Lagernadeln gruppiert sind.

Damit die Bearbeitung von Knochen und Gewebe eines Patienten vereinfacht werden kann, ist es günstig, wenn das distale Ende der Antriebswelle aus dem distalen Ende des Schafts herausragt. Beispielsweise kann so ein Fräser ausgebildet werden mit einem Fräswerkzeug oder Fräskopf, welches beziehungsweise welcher das distale Ende der Antriebswelle bildet beziehungsweise an diesem angeordnet oder gehalten ist.

Eine möglichst optimale Lagerung der Antriebswelle kann insbesondere dadurch erreicht werden, dass ein minimaler, von den Lagernadeln begrenzter Innendurchmesser des Nadellagers einem Außendurchmesser der Antriebswelle entspricht.

Die Montage des Instruments kann insbesondere dadurch vereinfacht werden, dass der Lagerkäfig einstückig ausgebildet ist.

Vorteilhafterweise ist der Lagerkäfig aus einem mindestens teilweise elastischen Material hergestellt. So kann er zur Montage so elastisch verformt werden, dass er beispielsweise in den oben beschriebenen Haltering eingeschoben werden kann. Nach Einbringen des Lagerkäfigs in den Haltering kann dieser dann in seine ursprüngliche Form zurückgehen und beispielsweise die über einen Sicherungsvorsprung in Form einer Kugel axial am Schaft gesichert werden. Insbesondere kann er beim Einsetzen in den Haltering bereits mit den Lagernadeln und gegebenenfalls Sicherungselementen in Form von Kugeln bestückt sein.

Günstig ist es, wenn der Lagerkäfig für jedes erste Sicherungselement eine Sicherungselementaufnahme umfasst, in welcher das Sicherungselement mit einem Halteglied gehalten ist. Eine solche Ausgestaltung ermöglicht es auf einfache Weise, die ersten Sicherungselemente am Lagerkäfig anzubringen beziehungsweise zu montieren. Beispielsweise kann das erste Sicherungselement in Form einer Kugel ausgebildet sein, die dann mittels des Halteglieds in einer entsprechenden Aufnahme, die insbesondere konisch geformt sein kann, gehalten wird.

Das Einbringen des ersten Sicherungselements in die Sicherungselementaufnahme kann insbesondere dadurch vereinfacht werden, dass das Halteglied von einer Montagestellung, in welcher das erste Sicherungselement in die Sicherungselementaufnahme einbringbar ist, in eine Sicherungsstellung bringbar ist, in welcher das erste Sicherungselement unverlierbar am Lagerkäfig gehalten ist. So lässt sich beispielsweise das erste Sicherungselement in der Montagestellung in die Sicherungselementaufnahme einsetzen und ist dann unverlierbar in der Sicherungsstellung am Lagerkäfig gehalten. Insbesondere ist es vorteilhaft, wenn das Halteglied elastisch federnd ausgebildet ist. Zum Beispiel kann so das erste Sicherungselement in die Sicherungselementaufnahme eingeschoben werden, wobei beim Einschieben das Halteglied von der Sicherungsstellung in die Montagestellung ausschwenken und nach endgültigem Einbringen des ersten Sicherungselements in die Sicherungselementaufnahme wieder in die Ausgangsstellung zurückschwenken und das erste Sicherungselement unverlierbar am Lagerkäfig halten kann.

Besonders einfach wird die Herstellung des Instruments, wenn das Halteglied in Form eines Haltestegs oder eines Haltearms ausgebildet ist.

Sowohl die Konstruktion als auch die Montage des Instruments lassen sich insbesondere dadurch weiter vereinfachen, dass der Lagerkäfig aus zwei identischen Lagerkäfigteilen ausgebildet ist.

Auf besonders einfache und schnelle Weise lassen sich die Lagerkäfigteile miteinander verbinden, wenn die Verbindungseinrichtung in Form einer Rastverbindungseinrichtung ausgebildet ist. Mit anderen Worten können so die Lagerkäfigteile miteinander in Eingriff gebracht werden und in einer Verbindungsstellung verrasten.

Günstig ist es, wenn die Verbindungseinrichtung erste und zweite Verbindungselemente umfasst, welche jeweils an einem der beiden Lagerkäfigteile angeordnet oder ausgebildet sind, welche ersten und zweiten Verbindungselemente in einer Trennstellung außer Eingriff stehen und in einer Verbindungsstellung miteinander in Eingriff stehen. Eine solche Verbindungseinrichtung lässt sich einfach herstellen und ermöglicht eine einfache und sichere Montage des Lagerkäfigs.

Vorzugsweise begrenzen jeweils ein erstes und ein zweites Verbindungselement eine Sicherungselementaufnahme mindestens teilweise. Die ersten und zweiten Verbindungselemente können so eine Doppelfunktion ausüben. Zum einen können sie die Sicherungselementaufnahme teilweise begrenzen, um beispielsweise so einen Sicherungsvorsprung in Form einer Kugel unverlierbar am Lagerkäfig zu halten. Zum anderen können Sie zur Verbindung der beiden Lagerkäfigteile dienen.

Gemäß einer weiteren bevorzugten Ausführungsform der Erfindung kann vorgesehen sein, dass das Nadellager mindestens ein Rückhalteelement umfasst zum Sichern der Lagernadeln am Lagerkäfig. Beispielsweise wenn Aussparungen am Lagerkäfig für die Lagernadeln in Richtung auf die Längsachse des Nadellagers hin geöffnet sind, so dass die Lagernadeln in Richtung auf die Längsachse aus dem Lagerkäfig herausfallen könnten, ermöglicht es das mindestens eine Rückhalteelement, dies gerade zu verhindern.

Günstig ist es, wenn das mindestens eine Rückhalteelement einen Teil einer inneren Lagerkäfigwand definiert, über die die Lagernadeln in radialer Richtung auf die Längsachse des Nadellagers hin weisend vorstehen. So können die Lagernadeln am Lagerkäfig durch das mindestens eine Rückhalteelement gehalten werden und gleichzeitig noch ihre Funktion als Lagerelemente für die Antriebswelle ausüben.

Besonders einfach und kostengünstig herstellen lässt sich das Instrument, wenn das mindestens eine Rückhalteelement einen Hülsenabschnitt definiert, welcher sich in Umfangsrichtung über mindestens 180° erstreckt. Beispielsweise kann der Hülsenabschnitt aus einem dünnen Blech geformt werden, welcher insbesondere Einschnitte oder Aussparung aufweisen kann, durch die die Lagernadeln mindestens teilweise hindurchreichen, um mit der Antriebswelle in Kontakt zu kommen.

Günstig ist es, wenn zwei Rückhalteelemente vorgesehen sind. Diese können insbesondere auch identisch ausgebildet sein. Selbstverständlich wäre es auch denkbar, zwei oder mehr Rückhalteelemente vorzusehen. Insbesondere dann, wenn jedoch der Lagerkäfig aus zwei identischen Lagerkäfigteilen ausgebildet ist, ist es vorteilhaft, wenn lediglich zwei Rückhalteelemente vorgesehen sind, die jeweils einem der beiden Lagerkäfigteile zugeordnet oder mit diesem verbindbar ausgebildet sind.

Insbesondere ist es vorteilhaft, die Rückhalteelemente identisch auszubilden. So kann ein Herstellungsaufwand des Instruments weiter minimiert werden.

Um zu verhindern, dass sich das mindestens eine Rückhalteelement in unerwünschter Weise vom Lagerkäfig lösen kann, ist es vorteilhaft, wenn das Instrument eine Kupplungseinrichtung zum Kuppeln des mindestens einen Rückhalteelements und des Lagerkäfigs umfasst.

Besonders einfach ausbilden lässt sich die Kupplungseinrichtung, wenn sie erste und zweite Kupplungselemente umfasst, welche einerseits am Lagerkäfig und andererseits am mindestens einen Rückhalteelement angeordnet oder ausgebildet sind und welche in einer Kupplungsstellung miteinander in Eingriff stehen.

Eine Montage des Lagerkäfigs lässt sich insbesondere dadurch vereinfachen, dass erste Kupplungselemente am mindestens einen Rückhalteelement von einer Einsetzstellung, in welcher das mindestens eine Rückhalteelement in den Lagerkäfig einbringbar ist, in die Kupplungsstellung bringbar sind. So kann das mindestens eine Rückhalteelement einfach in den Lagerkäfig eingesetzt werden, um die Lagernadeln an diesem zu sichern, und dann von der Einsetzstellung in die Kupplungsstellung überführt werden.

Vorzugsweise ist der Lagerkäfig aus einem Metall oder aus einem Kunststoff hergestellt. Insbesondere kann es sich dabei um ein sterilisierbares Material handeln. Bei dem Metall kann es sich insbesondere um einen korrosionsbeständigen Stahl handeln. Der Kunststoff kann beispielsweise ein dampfsterilisierbarer Kunststoff sein, also insbesondere Polyetheretherketon (PEEK) oder Polytetrafluorethylen (PTFE).

Um die Korrosionsbeständigkeit des Nadellagers insgesamt zu verbessern, ist es günstig, wenn die Lagernadeln und/oder die Sicherungselemente aus einem Metall hergestellt sind. Vorzugsweise sind sie aus einem korrosionsbeständigen Stahl ausgebildet. Sie können jedoch auch aus einem Hartmetall ausgebildet sein.

Vorteilhaft ist es, wenn die Lagernadeln und/oder die Sicherungselemente aus einer Keramik hergestellt sind. Insbesondere lässt sich so ein sehr abriebfestes Radiallager ausbilden.

Um einen Abrieb von aneinander anliegenden Teilen zu minimieren, ist es günstig, wenn die Lagernadeln oder die Sicherungselemente mit einer Hartstoffbeschichtung versehen sind. Insbesondere kann die Hartstoffbeschichtung auf korrosionsbeständigem Stahl oder auf einem Hartmetall aufgebracht sein.

Besonders günstig sind Hartstoffbeschichtungen, welche ein Metall-Nitrid enthalten. Insbesondere ist es vorteilhaft, Titannitrit oder Chromnitrit als Hartstoffbeschichtungen vorzusehen oder Hartstoffbeschichtungen, die diese Nitrite enthalten.

Die eingangs gestellte Aufgabe wird ferner bei einem chirurgischen Handstück der eingangs beschriebenen Art erfindungsgemäß dadurch gelöst, dass es eines der oben beschriebenen chirurgischen Instrumente umfasst.

Ein solches Handstück weist dann die oben im Zusammenhang mit bevorzugten Ausführungsformen chirurgischer Instrumente beschriebenen Vorteile auf. Das Handstück und das Instrument können insbesondere miteinander lösbar verbindbar sein, um so das Handstück mehrfach zu verwenden, auch wenn das chirurgische Instrument nur als Einweginstrument vorgesehen ist.

Des Weiteren wird die eingangs gestellte Aufgabe bei einem chirurgischen Antriebssystem der eingangs beschriebenen Art erfindungsgemäß dadurch gelöst, dass es mindestens eines der oben beschriebenen chirurgischen Instrumente umfasst.

Ein solches Antriebssystem weist dann die oben im Zusammenhang mit bevorzugten Ausführungsformen chirurgischer Instrumente beschriebenen Vorteile auf.

Die nachstehende Beschreibung bevorzugter Ausführungsformen der Erfindung dient im Zusammenhang mit den Zeichnungen der näheren Erläuterung. Es zeigen:
- Figur 1:: eine schematische Längsschnittansicht eines mit einem chirurgischen Handstück gekoppelten chirurgischen Instruments;

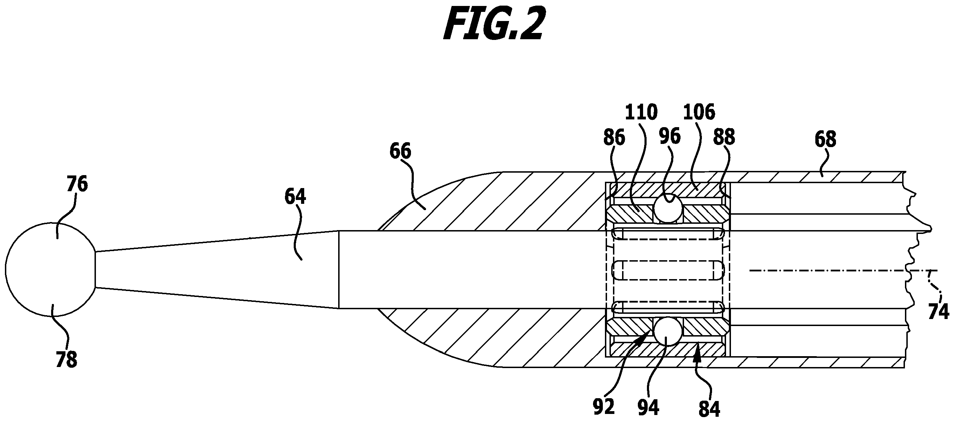

- Figur 2:: eine vergrößerte Ansicht eines distalen Endbereichs des Instruments aus Figur 1;
- Figur 3A:: eine perspektivische Ansicht eines ersten Ausführungsbeispiels des in Figur 2 dargestellten Radiallagers;
- Figur 3B:: eine Explosionsdarstellung der in Figur 3A dargestellten Anordnung;
- Figur 3C:: eine Querschnittsansicht der in Figur 3A dargestellten Anordnung;
- Figur 3D:: eine Schnittansicht längs Linie 3D-3D in Figur 3C;
- Figur 4A:: eine Explosionsdarstellung eines zweiten Ausführungsbeispiels eines Radiallagers;
- Figur 4B:: eine Schnittansicht längs Linie 4B-4B in Figur 4C;
- Figur 4C:: eine Schnittansicht längs Linie 4C-4C in Figur 4B;
- Figur 4D:: eine perspektivische Ansicht des Lagerkäfigs der in Figur 4A dargestellten Anordnung;
- Figur 5A:: eine Explosionsdarstellung eines dritten Ausführungsbeispiels eines Radiallagers;
- Figur 5B:: eine Querschnittsansicht der in Figur 5A dargestellten Anordnung;
- Figur 5C:: eine Seitenansicht des in Figur 5B dargestellten Anordnung in Richtung des Pfeils 5C;
- Figur 5D:: eine teilweise durchbrochene Explosionsdarstellung des Lagerkäfigs der in Figur 5A dargestellten Anordnung in der Trennstellung;
- Figur 5E:: eine vergrößerte, teilweise durchbrochene Darstellung eines der beiden identischen, in Figur 5D dargestellten Lagerkäfigteile;
- Figur 6A:: eine Explosionsdarstellung eines vierten Ausführungsbeispiels eines Radiallagers;
- Figur 6B:: eine Schnittansicht längs Linie 6B-6B in Figur 6C;
- Figur 6C:: eine Seitenansicht der in Figur 6B dargestellten Anordnung in Richtung des Pfeils 6C;
- Figur 6D:: eine Schnittansicht längs Linie 6D-6D in Figur 6C;
- Figur 6E:: eine teilweise durchbrochene perspektivische Ansicht des Lagerkäfigs der in Figur 6A dargestellten Anordnung;
- Figur 6F:: eine teilweise durchbrochene Explosionsdarstellung des in Figur 6E dargestellten Lagerkäfigs; und
- Figur 7:: eine schematische Gesamtdarstellung eines chirurgischen Antriebssystems.

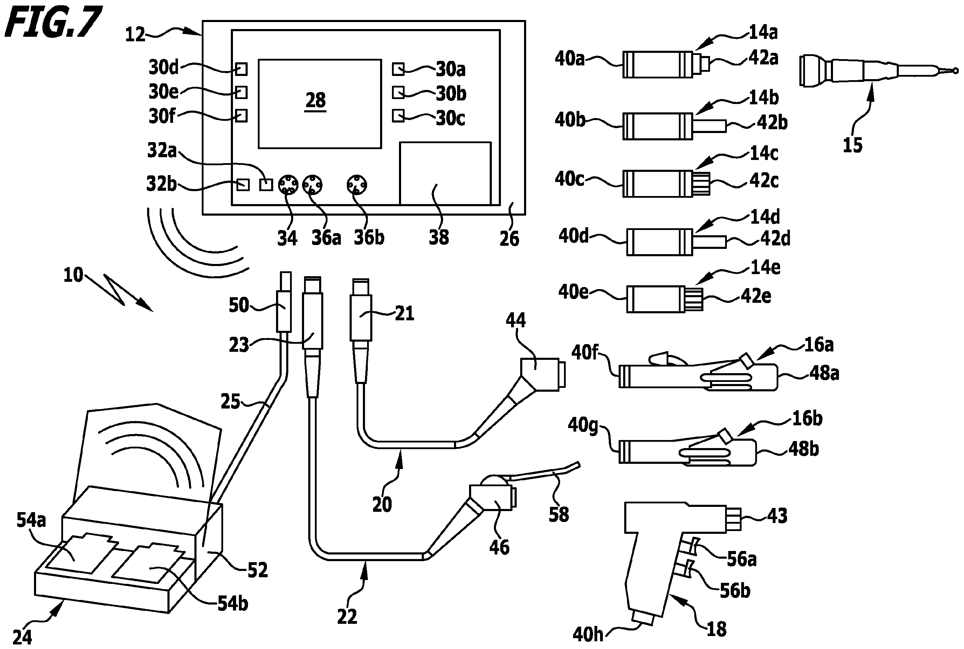

In Figur 7 ist schematisch ein insgesamt mit dem Bezugszeichen 10 versehenes chirurgisches Antriebssystem dargestellt, umfassend eine Steuer- und/oder Regelungseinrichtung in Form eines Steuergeräts 12, fünf Handstücke 14a bis 14e, zwei Shaverhandstücke 16a und 16b, ein Pistolenhandstück 18, zwei Versorgungsleitungen in Form von Anschlusskabeln 20 und 22 sowie eine Fußsteuerung 24. Alle genannten Handstücke umfassen einen integrierten Elektromotor als Antrieb und bilden so Antriebseinheiten.

Das Steuergerät 12 umfasst einen in einem Gehäuse 26 angeordneten flachen Bildschirm 28 in Form eines Touchscreens. Zu beiden Seiten des Bildschirms 28 sind je drei Bedienelemente 30a bis 30c beziehungsweise 30d bis 30f angeordnet.

Zwei Schalter 32a und 32b sind unterhalb des Bildschirms 28 auf einer Linie angeordnet mit einer Anschlussbuchse 34 zum Anschluss der Fußsteuerung 24 über ein optionales Anschlusskabel 25 und mit zwei Anschlussbuchsen 36a und 36b zum Anschluss der Anschlusskabel 20 und 22, mit denen die Handstücke mit dem Steuergerät 12 verbunden werden können. Optional kann außerdem ein Anschluss 38 für ein Fluidsystem zur Zufuhr und Abfuhr von Fluiden aus einem Operationsbereich vorgesehen sein, beispielsweise auch zur Versorgung von Spül- oder Absaugkanälen an mit den Handstücken 14, den Shaverhandstücken 16 oder dem Pistolenhandstück 18 verbindbaren, nicht dargestellten Getriebeeinheiten oder Werkzeugen, mit welchen zusammen die Handstücke chirurgische Instrumente des Antriebssystems 10 bilden.

Die Handstücke 14a bis 14e umfassen jeweils eine Kabelkupplung 40a bis 40e, die mit einem Kupplungsstück 44 des Anschlusskabels 20 oder einem Kupplungsstück 46 des Anschlusskabels 22 beliebig verbindbar sind. Ebenso weisen die beiden Shaverhandstücke 16a und 16b sowie das Pistolenhandstück 18 jeweils eine Kabelkupplung 40f, 40g beziehungsweise 40h auf, die mit einem der beiden Kupplungsstücke 44 oder 46 verbindbar sind.

An ihrem jeweils anderen Ende sind die Handstücke 14a bis 14e mit Getriebe- oder Werkzeugkupplungen ausgestattet, welche Werkzeugkupplungseinrichtungen 42a bis 42e definieren, auf die nicht dargestellte Getriebeeinheiten, beispielsweise ausgestattet mit Bohrern, Sägeblättern oder dergleichen angekuppelt und durch die Handstücke 14a bis 14e angetrieben werden können. Je nach Ausgestaltung können die Handstücke 14a bis 14e auch direkt mit chirurgischen Instrumenten 15, beispielsweise Fräsern, Bohrern oder Sägeblättern, bestückt werden.

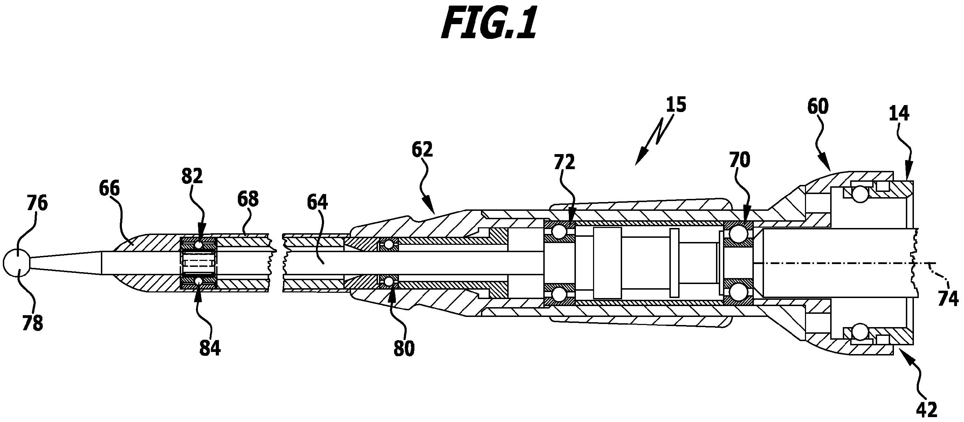

Die Handstücke 14a bis 14e sind vorzugsweise sensorlos ausgebildet, das heißt sie weisen keine Sensoren auf, um eine Drehzahl der Handstücke 14a bis 14e während des Betriebs zu bestimmen. Die Handstücke des Antriebssystems 10 unterscheiden sich nicht nur, wie in Figur 1 schematisch dargestellt, äußerlich, sondern auch hinsichtlich ihres inneren Aufbaus. Dies bedeutet, dass die in den Handstücke 14a bis 14e verbauten Elektromotoren unterschiedlichen Typs sein und sich beispielsweise in ihren Kenngrößen, wie zum Beispiel Minimaldrehzahl, Maximaldrehzahl, Maximalstrom und Maximaldrehmoment, unterscheiden können. Zudem können, wie bei den beiden Shaverhandstücken 16a und 16b, Getriebe integriert sein, welche optional auch in an die Handstücke 14a bis 14e sowie an das Pistolenhandstück 18 ankoppelbare Getriebeeinheiten integriert sein können. Die Getriebeeinheiten können je nach Ausgestaltung auch selbst zusätzlich mit unterschiedlichen chirurgischen Instrumenten 15 in Form chirurgischer Werkzeuge bestückt werden.

Des Weiteren umfassen die Shaverhandstücke 16a und 16b jeweils eine Shaverkupplung 48a beziehungsweise 48b zum Anschluss eines Shaveraufsatzes, beispielsweise zur Anwendung in der Arthroskopie.

Die Anschlusskabel 20 und 22 sind zum Verbinden mit dem Steuergerät mit Kupplungen 21 und 23 versehen, über die sie mit den Anschlussbuchsen 36a und 36b verbindbar sind.

Die Fußsteuerung 24 steht über eine drahtlose Datenübertragungseinrichtung mit dem Steuergerät 12 in Verbindung, beispielsweise über ein Infrarot- oder Funkübertragungssystem. Optional ist auch eine Verbindung der Fußsteuerung 24 über ein mit der Anschlussbuchse 34 verbindbares Kupplungsstück 50 des Anschlusskabels 25 möglich. An einem Gehäuse 52 der Fußsteuerung 24 sind zwei fußbetätigbare Schalter 54a und 54b angeordnet, über die insbesondere ein Links- beziehungsweise Rechtslauf der Handstücke geregelt werden kann.

Das Pistolenhandstück 18 ist mit zwei Gebern 56 ausgestattet, wobei der Geber 56a beispielsweise zur Aktivierung eines Motorrechtslaufes, der Geber 56b zur Aktivierung eines Motorlinkslaufes vorgesehen sein können.

Die Anschlusskabel 20 und 22 unterscheiden sich dadurch, dass am Anschlusskabel 22, anders als am Anschlusskabel 20, ein Betätigungshebel 58 vorgesehen ist, mit dem eine Bedienperson einen Motorbetrieb eines Handstücks 14, eines Shaverhandstücks 16 oder des Pistolenhandstücks 18 aktivieren kann. Der Betätigungshebel 58 hat die Funktion eines Drehzahlgebers, mit dem eine Drehzahl des Motors von einer Bedienperson vorgegeben werden kann.

In Figur 1 ist der Aufbau eines chirurgischen Instruments 15 schematisch dargestellt. Ein proximales Ende des Instruments 15 ist in Form Kupplungsabschnitts 60 ausgebildet, welcher mit einer Werkzeugkupplungseinrichtung 42 des Handstücks 14 koppelbar ist. Das Instrument 15 umfasst einen langgestreckten hohlen Schaft 62, welcher sich distalseitig der Werkzeugkupplungseinrichtung 42 erstreckt und in welchem eine Antriebswelle 64 rotierbar gelagert ist. Der Schaft 62 kann mehrteilig ausgebildet sein und sich, wie bei dem in Figur 1 dargestellten Ausführungsbeispiel, in Richtung auf ein distales Ende 66 hin ein oder mehrfach im Außendurchmesser verjüngen. Ein distaler Schaftabschnitt 68, welcher sich bis zum Ende 66 hin erstreckt, weist einen minimalen Außendurchmesser auf. Dieser kann insbesondere kleiner als 5 mm sein.

Zur Lagerung der Antriebswelle 64 im Schaft 62 sind etwas distalseitig des Kupplungsabschnitts 60 im Inneren des Schafts 62 zwei Radiallager in Form von Kugellagern 70 und 72 in axialer Richtung versetzt, also bezogen auf eine Längsachse 74 des Schafts 62, angeordnet. Distalseitig des noch im proximalen Endbereich des Schafts 62 angeordneten Kugellagers 72 weist die Antriebswelle 64 einen reduzierten Außendurchmesser auf, welcher bis über das Ende 66 hinaus konstant bleibt. Ein distales Ende der Antriebswelle 64 bildet ein Werkzeugelement 76, beispielsweise in Form eines kugeligen Fräskopfs 78, welcher drehfest mit der Antriebswelle 64 verbunden oder einstückig mit dieser ausgebildet ist.

Etwas distalseitig des Kugellagers 72, jedoch proximalseitig bezogen auf den Übergang zum Schaftabschnitt 68, ist im Inneren des Schafts 62 ein weiteres Kugellager 80 angeordnet, welches zur Lagerung der Antriebswelle 64 im Schaft 62 dient.

Der Schaftabschnitt 68 kann relativ lang sein, so dass er in Figur 1 schematisch unterbrochen dargestellt ist. Dies hat zur Folge, dass im Bereich des distalen Endes 66 beim Einsatz des Instruments 15 hohe Kräfte, insbesondere Querkräfte bezogen auf die Längsachse 74, auf das Werkzeugelement 76 und damit auf die Antriebswelle 64 wirken. Um diese Kräfte mit möglichst geringer Reibung aufnehmen und die Antriebswelle 64 relativ zum Schaft 62 abstützen zu können, ist etwas proximalseitig des Endes 66 im Inneren des Schafts 62 ein weiteres Radiallager 82 vorgesehen, jedoch nicht in Form eines Kugellagers, sondern in Form eines Nadellagers 84. Das Nadellager 84 stützt sich distalseitig an einer in proximaler Richtung weisenden Ringfläche 86 ab, die durch eine einstufige Verjüngung des Innendurchmessers des Schafts 62 ausgebildet wird. Proximalseitig stützt sich das Nadellager 84 an einer weiteren Ringfläche 88 ab, welche ein distales Ende einer in den Schaft 62 eingesetzten und in axialer Richtung fixierten Klemmhülse 90 definiert.

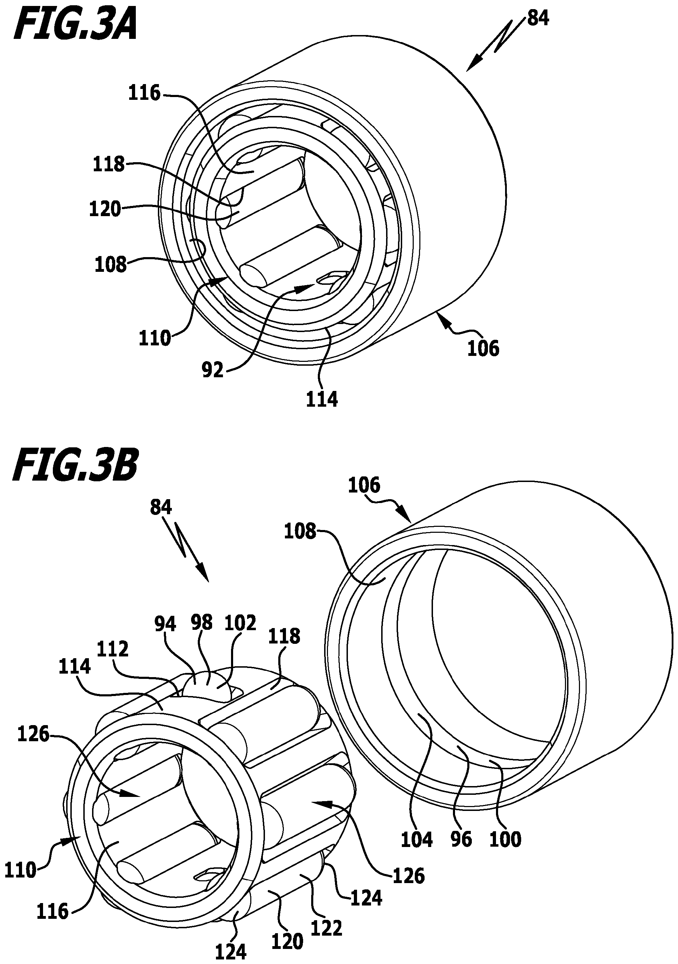

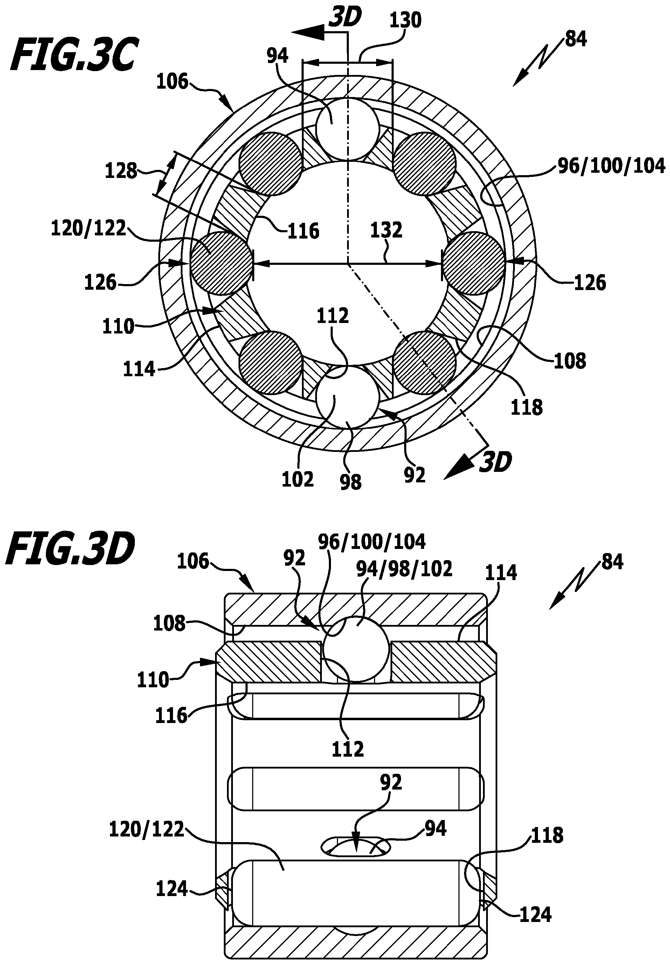

Ein Aufbau des Nadellagers 84 wird nachfolgend in Verbindung mit den Figuren 3A bis 3D näher erläutert.

Das eigentliche Nadellager 84 ist mittels einer Nadellagersicherungseinrichtung 92 axial am Schaft gesichert. Die Nadellagersicherungseinrichtung 92 umfasst erste Sicherungselemente 94 und ein zweites, zu diesen korrespondierendes Sicherungselement 96. Die Sicherungselemente 94, 96 stehen quer zur Längsachse 74 in einer Sicherungsstellung miteinander in Eingriff. Die ersten Sicherungselemente 94 sind in Form von Sicherungsvorsprüngen 98 ausgebildet, welche in eine durch das zweite Sicherungselement 96 definierte Sicherungsausnehmung 100 eingreifen. Die Sicherungsvorsprünge 98 sind ausgebildet in Form von frei rotierbar gelagerten Kugeln 102, die Sicherungsausnehmung 100 in Form einer zu den Kugeln 102 im Querschnitt korrespondierenden Ringnut 104. Die Ringnut 104 wiederum ist an einem hülsenförmigen Haltering 106 ausgebildet, und zwar in einer inneren Wandfläche 108 desselben.

Die Kugeln 102 sind an einem Lagerkäfig 110 in speziell für diese geformten Sicherungselementaufnahmen 112 gehalten. Bei diesen handelt es sich um Durchbrechungen des hülsenförmigen Lagerkäfigs 110, welche sich im innern Querschnitt in Richtung auf die Längsachse 74 hin näherungsweise konisch verjüngen. Durch entsprechende Bemessung der Sicherungselementaufnahmen 112 ragen die Kugeln 102 über eine Außenfläche 114 des Lagerkäfigs 110 vor, nicht jedoch über dessen konzentrisch zur Längsachse 74 verlaufende Innenfläche 116.

Die beiden Sicherungselementaufnahmen 112 sind einander diametral gegenüberliegend, das heißt um 180° in Umfangsrichtung versetzt, am Lagerkäfig 110 ausgebildet. Zwischen den beiden Sicherungselementaufnahmen 112 sind am Lagerkäfig 110 jeweils drei Nadelaufnahmen 118 zur Aufnahme jeweils einer Lagernadel 120 ausgebildet. Es handelt sich bei den Nadelaufnahmen 118 um langlochartige Durchbrechungen des Lagerkäfigs 110, deren innerer Querschnitt sich in radialer Richtung mit zunehmendem Abstand von der Längsachse 74 erweitert. Die Lagernadeln 120 sind in Form massiver Zylinderbolzen 122 ausgebildet, welche jeweils halbkugelige, voneinander weg weisende Enden 124 aufweisen. Die Nadelaufnahmen 118 sind so bemessen, dass die Lagernadeln 120 etwas über die Innenfläche 116 in Richtung auf die Längsachse 74 vorstehen und somit direkt mit der Antriebswelle 64 in Kontakt kommen können. Diese kann sich dann, anders als bei einem Kugellager, nicht nur punktförmig, sondern linienförmig abstützen, und zwar an allen sechs Lagernadeln 120.

Jeweils drei Lagernadeln 120 definieren eine Nadelgruppe 126. Ein Abstand 128 zwischen benachbarten Lagernadeln 120 einer Nadelgruppe 126 ist kleiner als ein Abstand 130 zwischen zwei Lagernadeln 120 benachbarter Nadelgruppen 126. Die Kugeln 102 sind am Lagerkäfig 110 im Bereich zwischen den beiden Nadelgruppen 126 ausgebildet.

Der Lagerkäfig 110 ist einstückig ausgebildet, wobei er jedoch so elastisch ist, dass er zum Einsetzen nach Bestücken mit den Kugeln 102 sowie den Lagernadeln 120 zum Einsetzen in den Haltering 106 etwas verformt werden kann, bis die Kugeln 102 in die Ringnut 104 eingreifen. Sobald dies der Fall ist, geht der Lagerkäfig 110 wieder in seine ursprüngliche Form zurück und wird so über die Kugeln 102 axial am Schaft 62 gesichert. Um die gewünschte Elastizität des Lagerkäfigs 110 zu erreichen, ist dieser aus einem Material hergestellt, welches bei entsprechender Dimensionierung Kraftbeaufschlagung elastisch verformbar ist. Insbesondere kann der Lagerkäfig 110 aus einem Kunststoff hergestellt sein.

Beim Nadellager 84 entspricht die Zahl der Lagernadeln 120 einem ganzzahligen Vielfachen der Zahl sowohl der ersten Sicherungselemente 94 als auch der zweiten Sicherungselemente 96. Insgesamt gibt es dreimal so viele Lagernadeln 120 wie erste Sicherungselemente und sechsmal so viele Lagernadeln 120 wie zweite Sicherungselemente 94.

Des Weiteren entspricht ein minimaler, von den Lagernadeln 120 begrenzter Innendurchmesser 132 des Nadellagers 84 einem Außendurchmesser der Antriebswelle 64 beziehungsweise weist das allenfalls hierfür erforderliche Spiel auf, um eine möglichst reibungsfreie Rotation der Antriebswelle 64 im Schaft 82 zu gestatten.

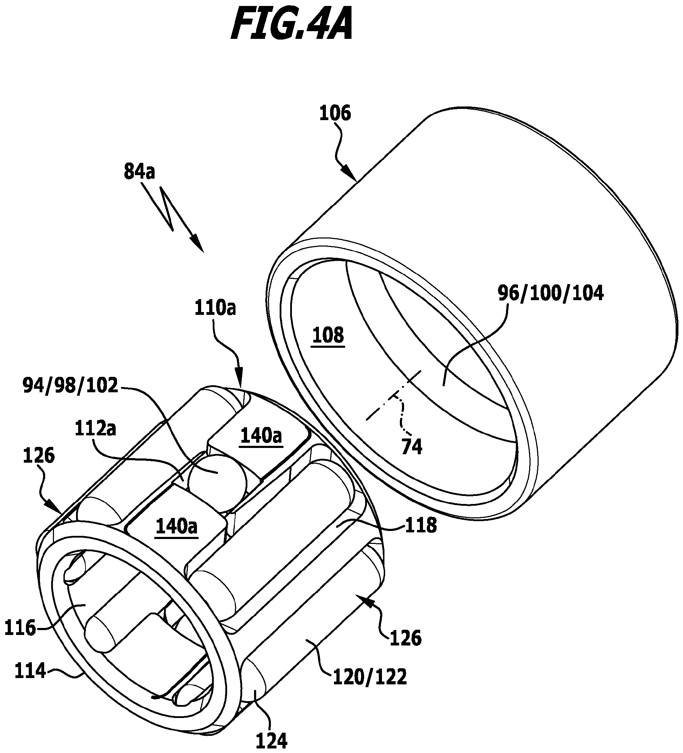

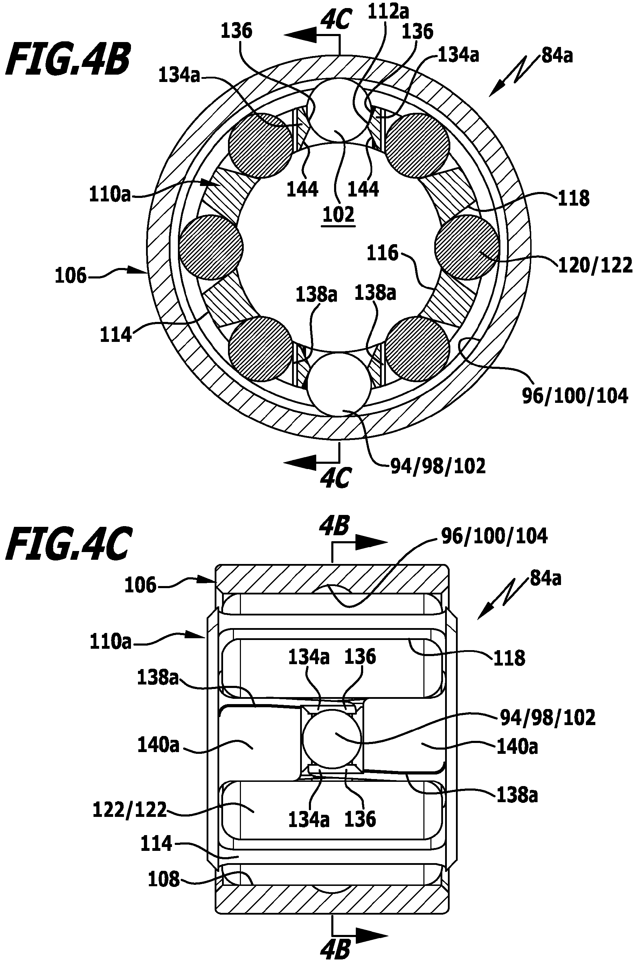

Ein weiteres Ausführungsbeispiel eines Nadellagers ist schematisch in den Figuren 4A bis 4D dargestellt und insgesamt mit dem Bezugszeichen 84a bezeichnet. Es wird mittels des Halterings 106 in der oben beschriebenen Weise am Schaft 62 axial gesichert.

Das Nadellager 84a unterscheidet sich lediglich im Aufbau des Lagerkäfigs 110a vom Nadellager 84. Nachfolgend werden daher nur diejenigen Elemente und Teile, die sich beim Nadellager 84a vom Nadellager 84 unterscheiden, jeweils mit identischen Bezugszeichen, jedoch nachgestelltem Buchstaben a bezeichnet. Entsprechend gilt dies für die nachfolgend ebenfalls beschriebenen weiteren Ausführungsbeispiele, bei denen Bezugszeichen mit nachgestellten Buchstaben b beziehungsweise c versehen sind.

Zahl, Anordnung und Ausgestaltung der Lagernadeln 120 sowie der Sicherungsvorsprünge 98 stimmen beim Nadellager 84a mit denjenigen des Nadellagers 84 überein. Ein Unterschied besteht lediglich in der Ausgestaltung der Sicherungselementaufnahmen 112a. Diese werden in Umfangsrichtung begrenzt durch zwei parallel zueinander und zur Längsachse 74 verlaufende Halteglieder 134, welche zwei gegeneinander geneigte Lagerflächen 136 für die Kugeln 102 aufweisen. Die Halteglieder 134 sind jeweils in Umfangsrichtung durch einen schmalen Schlitz 138 von einem im Wesentlichen quaderförmigen Begrenzungsvorsprung 140 getrennt und weisen auf den jeweils anderen Begrenzungsvorsprung 140 hin. Die Halteglieder 134 können in Umfangsrichtung somit etwas ausfedern, wodurch sich der Schlitz 138 etwas verbreitern kann. Dies ermöglicht es auf einfache Weise, die Kugeln 102 in die Sicherungselementaufnahme 112 einzusetzen.

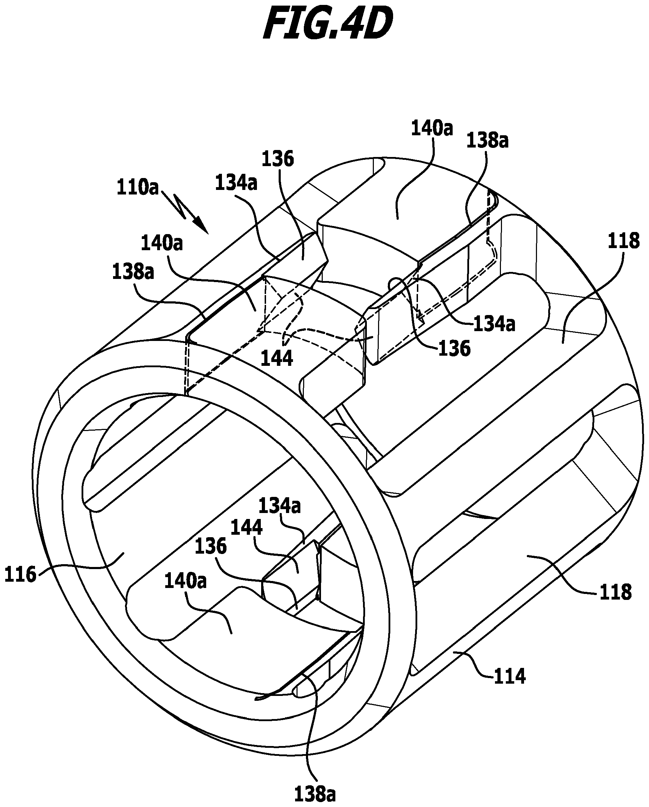

Das Vorsehen der Haltearme 142 definierenden Halteglieder 134 ermöglicht es, den Lagerkäfig 110 aus einem im Wesentlichen inelastischen Material oder so steif auszubilden, dass der Lagerkäfig 110 nicht mit daran gelagerten Lagernadeln 120 und Kugeln 102 in den Haltering 106 eingesetzt werden kann. Zur Montage des Nadellagers 84a im Haltering 106 wird der Lagerkäfig 110a zunächst mit den Lagernadeln 120 bestückt und in den Haltering 106 eingesetzt. Anschließend werden von innen die Kugeln 102 gegen zueinander geneigte Aufgleitflächen 144 der Haltearme 142 gedrückt, so dass sich diese in Umfangsrichtung etwas aufspreizen und die Kugeln 102 in die Sicherungselementaufnahmen 112 hineingleiten können. Sobald die Kugeln 102 durch die von den Haltegliedern 134 und den Begrenzungsvorsprüngen 140 begrenzte Durchbrechung hindurchgelangt sind, können die Halteglieder 134 wiederum von ihrer gespreizten Stellung, die auch als Montagestellung bezeichnet wird, wieder in ihre Ausgangsstellung, wie sie beispielsweise schematisch in Figur 4D dargestellt ist, zurückschwenken. Letztgenannte Stellung wird auch als Sicherungsstellung bezeichnet, in welcher die Kugeln 102 in den Sicherungselementaufnahmen 112 gesichert sind.

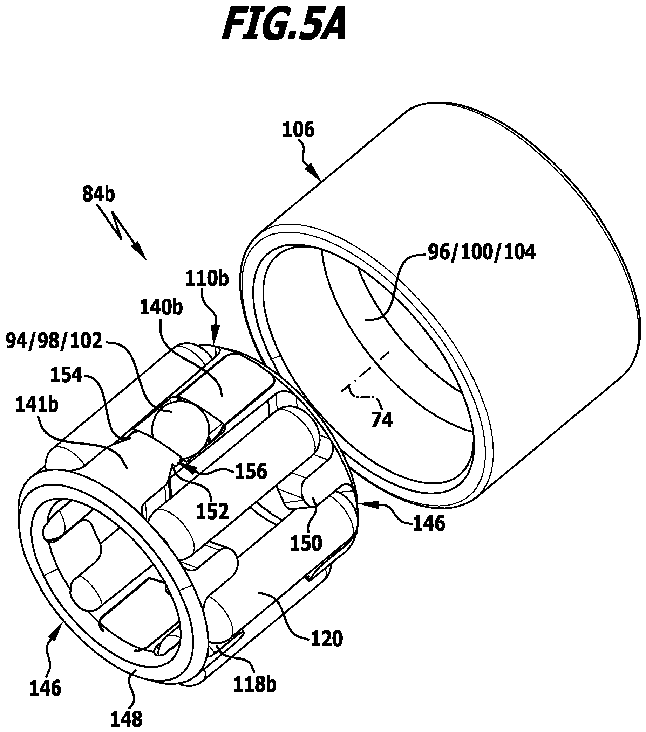

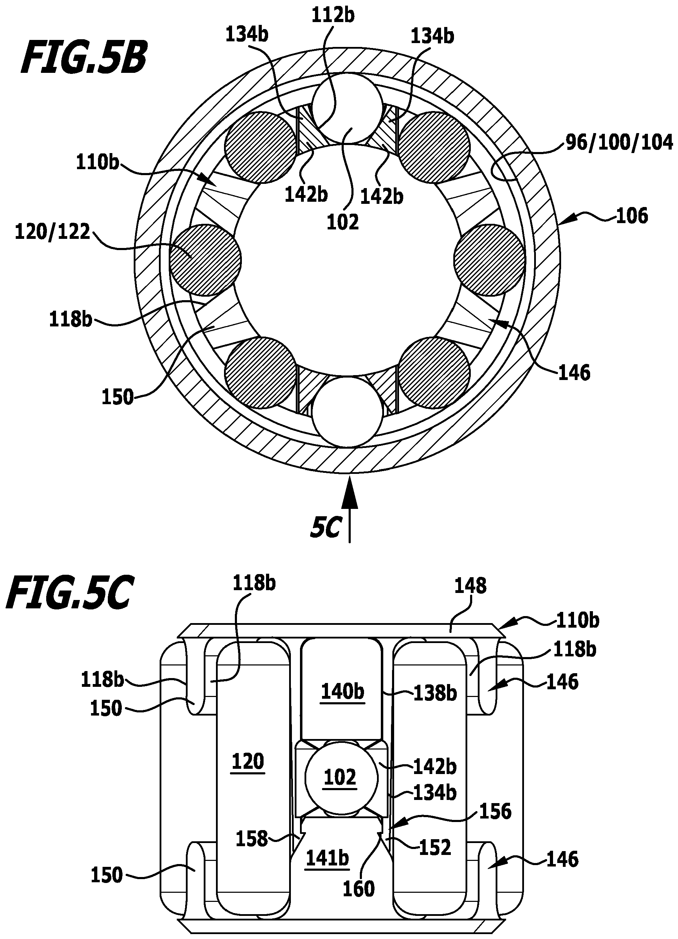

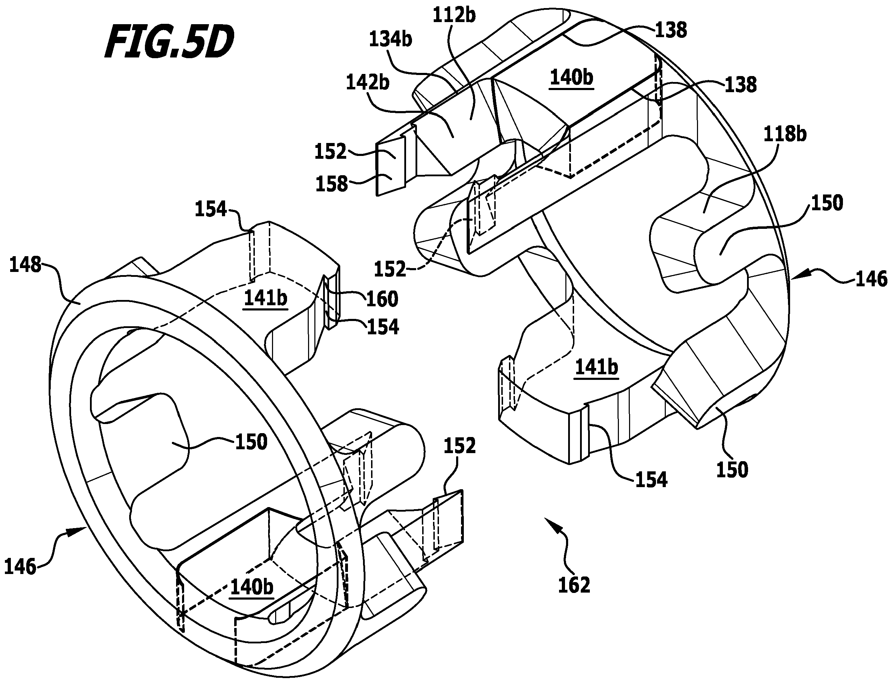

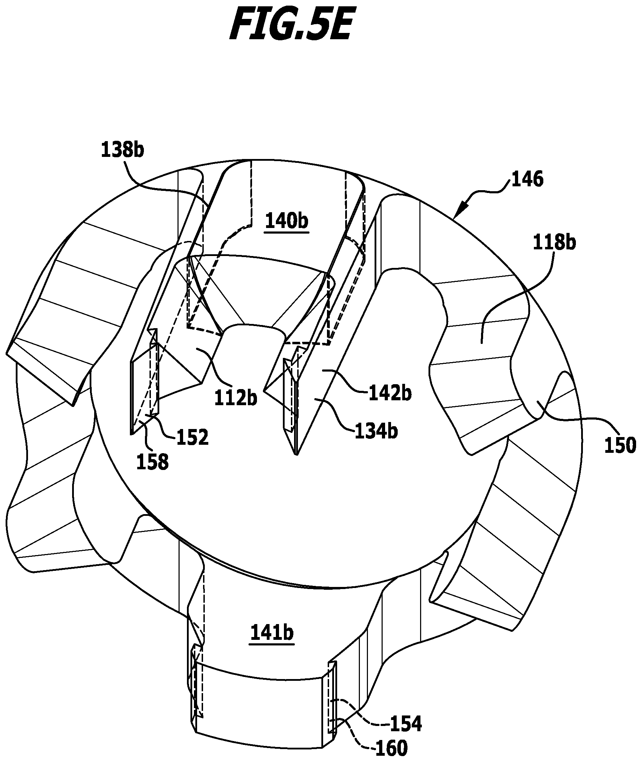

Ein weiteres Ausführungsbeispiel eines Nadellagers ist in den Figuren 5A bis 5E schematisch dargestellt und insgesamt mit dem Bezugszeichen 84b bezeichnet. Es wird in der oben beschriebenen Weise mittels des Halterings 106 axial am Schaft 62 gesichert.

Das Nadellager 84b unterscheidet sich vom Nadellager 84a lediglich durch die Ausgestaltung des Lagerkäfigs 110b. Dieser ist zweiteilig ausgebildet und umfasst zwei identische Lagerkäfigteile 146. Jeder Lagerkäfigteil 146 umfasst einen Ring 148, welcher die Längsachse 74 konzentrisch umgibt. Vom Ring 148 stehen im Wesentlichen parallel zur Längsachse 74 jeweils sechs Vorsprünge ab, von denen vier Vorsprünge 150 identisch ausgebildet sind. Diese erstrecken sich jeweils über etwa ein Viertel einer Gesamtlänge des Lagerkäfigs 110b parallel zur Längsachse 74. Sie begrenzen jeweils Nadelaufnahmen 118b. Die Vorsprünge 150 sind jeweils paarweise angeordnet, wobei zwischen jeweils einem Paar von Vorsprüngen 150 ein im Wesentlichen quaderförmiger Begrenzungsvorsprung 140b beziehungsweise 141b ausgebildet ist. Der Begrenzungsvorsprung 140b ist durch Schlitze 138b von Haltegliedern 134b getrennt, welche in Umfangsrichtung eine Sicherungselementaufnahme 112b begrenzen.

Freie Enden der in Form von Haltearmen 142b ausgebildeten Halteglieder 134b sind in Form erster Verbindungselemente 152 ausgebildet, welche korrespondierend zu zweiten Verbindungselementen 154 am Begrenzungsvorsprung 141b ausgebildet sind. Die ersten und zweiten Verbindungselemente 152 und 154 bilden Teile einer Verbindungseinrichtung 156 zum Verbinden der beiden Lagerkäfigteile 146 miteinander in einer Verbindungsstellung. Die ersten Verbindungselemente 152 sind in Form von Rastvorsprüngen 158 ausgebildet, welche in korrespondierende Rastausnehmungen 160, die durch die zweiten Verbindungselemente 154 definiert werden, in der Verbindungsstellung eingreifen können. Die Rastvorsprünge 158 und die Rastausnehmungen 160 definieren jeweils Kanten, die quer zur Längsachse 74 verlaufen und so ein versehentliches Lösen der Lagerkäfigteile 146 voneinander durch lediglich eine Zugbeanspruchung parallel zur Längsachse 74 unmöglich machen.

Die Begrenzungsvorsprünge 140b und 141b sind in Umfangsrichtung um 180° versetzt zueinander angeordnet, so dass die ersten Verbindungselemente 152 eines Lagerkäfigteils 146 mit jeweils zweiten Verbindungselementen 154 des anderen Lagerkäfigteils 146 in Eingriff gebracht werden können.

Die Lagerkäfigteile 146 können bis auf die Haltearme 142b im Wesentlichen steif und unverformbar ausgebildet sein. Zur Montage wird ein Lagerkäfigteil 146 in den Haltering 106 eingesetzt. Die sechs Lagernadeln 120 sowie die beiden Kugeln 102 werden in diesen Lagerkäfigteil 146 eingelegt. Dann wird der zweite Lagerkäfigteil 146 von der anderen Seite in den Haltering 106 eingeschoben, bis die eine Rastverbindungseinrichtung 162 ausbildenden Rastvorsprünge 158 und Rastausnehmungen 160 ineinander eingreifen und verrasten können. Die Lagerkäfigteile 146 nehmen dann die Verbindungsstellung ein, so dass der Lagerkäfig 110 in der oben beschriebenen Weise mittels der ersten Sicherungselemente 94 axial über den Halterring 106 am Schaft 62 gesichert ist.

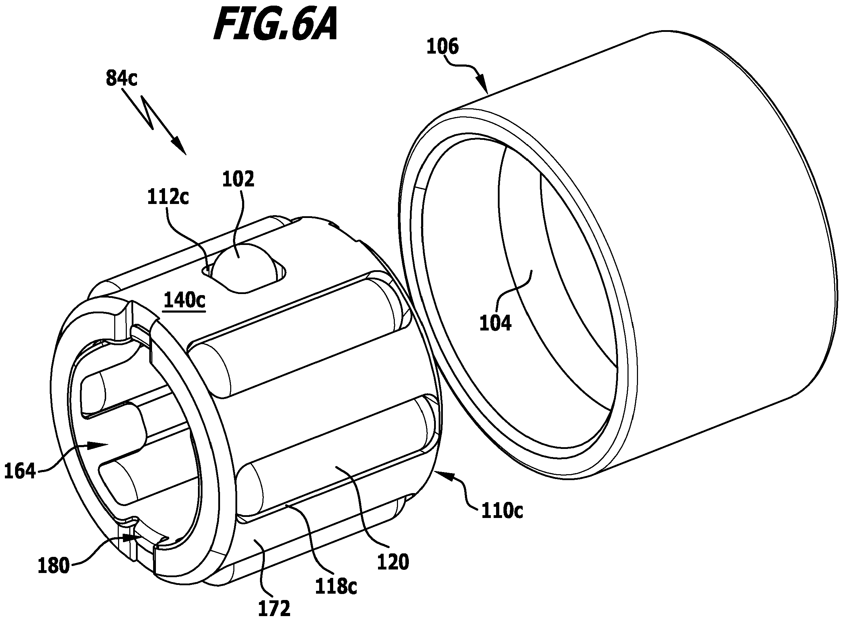

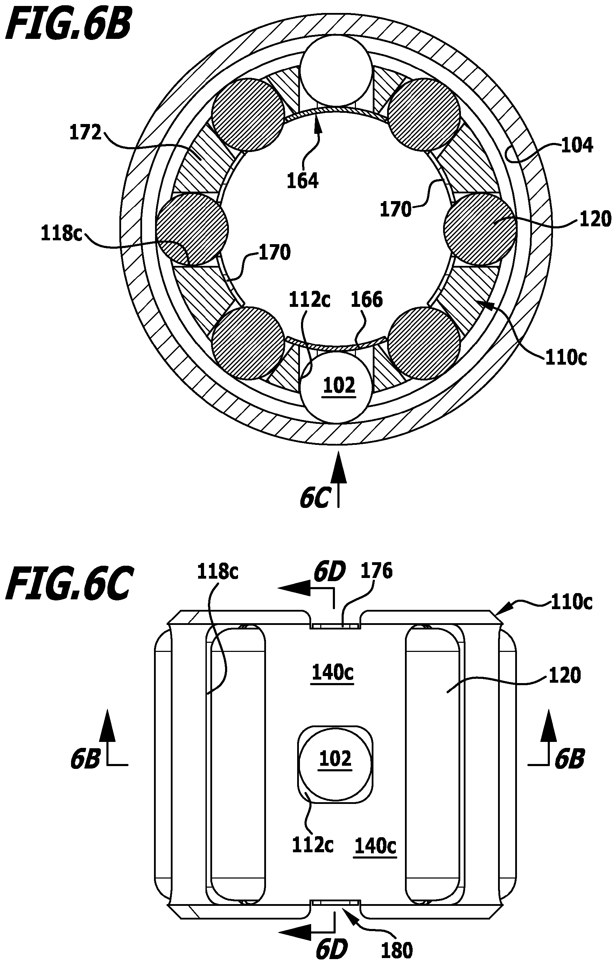

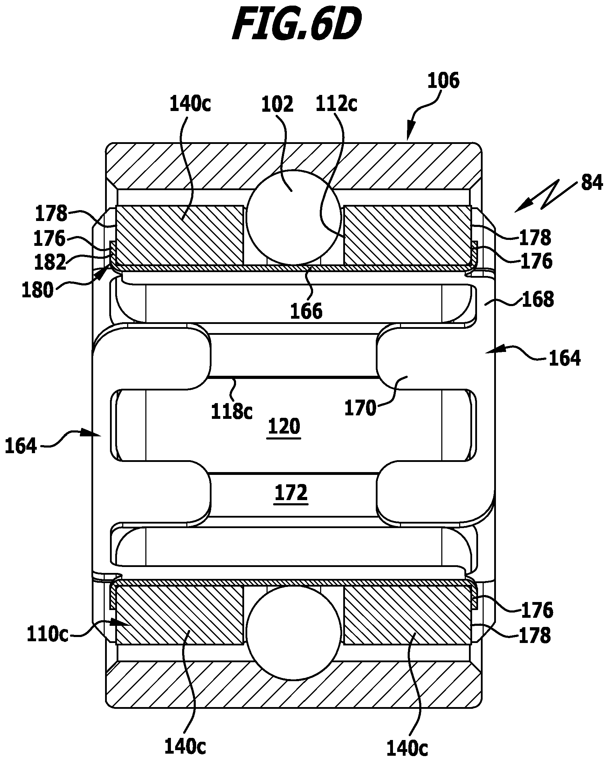

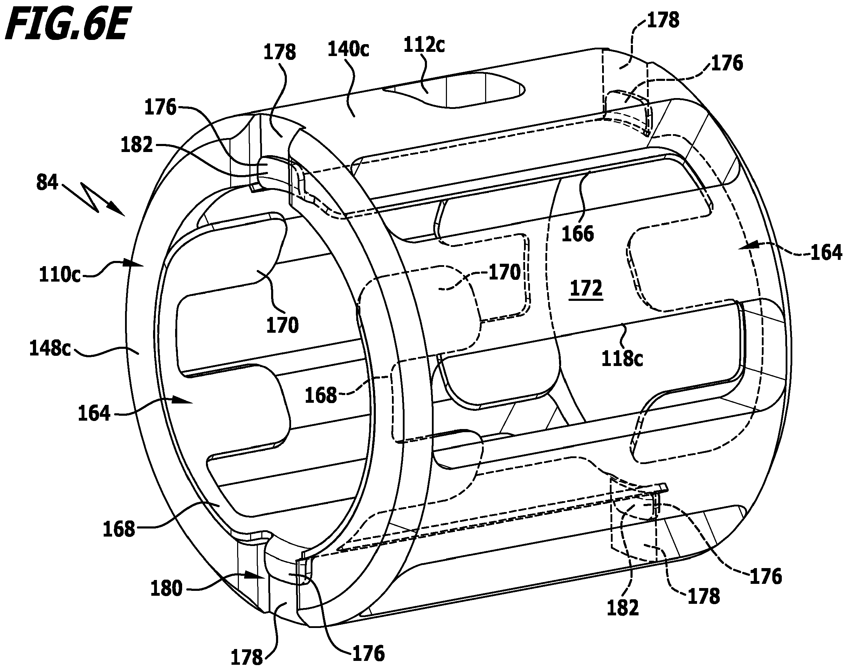

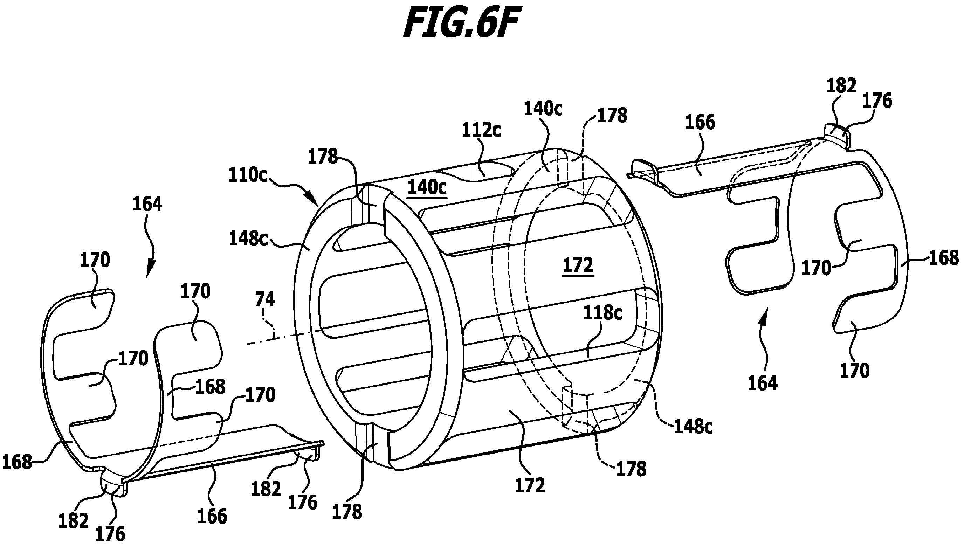

Ein weiteres Ausführungsführungsbeispiel eines Nadellagers ist schematisch in den Figuren 6A bis 6F insgesamt mit dem Bezugszeichen 84c bezeichnet. Es kann im Zusammenwirken mit dem Haltering 106 axial am Schaft 62 gesichert werden.

Das Nadellager 84c umfasst einen Lagerkäfig 110c, welcher einstückig ausgebildet ist. Er definiert Sicherungselementaufnahmen 112c für jeweils eine Kugel 102 sowie Nadelaufnahmen 118c für jeweils eine der sechs Lagernadeln 120. Sowohl die Sicherungselementaufnahmen 112c als auch die Nadelaufnahmen 118c erweitern sich im Wesentlichen konisch in Richtung auf die Längsachse 74 hin.

Um ein Durchfallen beziehungsweise Herausfallen sowohl der Kugel 102 als auch der Lagernadeln 120 in Richtung auf die Längsachse 120 zu verhindern, sind zwei identische Rückhaltelemente 164 vorgesehen. Diese sind jeweils aus einem flachen Metallstreifen geformt und bilden einen Ausschnitt einer zylindrischen Hülse. Die Rückhalteelemente 164 sind zudem symmetrisch zu einer die Längsachse 74 enthaltenden Symmetrieebene ausgebildet. Sie umfassen einen Mittelstreifen 166, welcher von innen die aufeinander zu weisenden Begrenzungsvorsprünge 140c und damit auch die Sicherungselementaufnahme 112 überdeckt. In Umfangsrichtung erstrecken sich von einem Ende des Mittelstreifens 166 zwei Steg 168 in entgegengesetzten Richtungen weg, welche jeweils zwei Rückhalteblättchen 170 tragen. Diese überdecken jeweils einen der die Nadelaufnahmen 118c in Umfangsrichtung seitlich begrenzenden Stege 172 teilweise, sind jedoch etwas breiter als diese in Umfangsrichtung, so dass sie ebenso wie die Mittelstreifen 166 seitlich etwas die Nadelaufnahmen 118 überdecken. Sie definieren zwischen sich Öffnungen 174, die hinreichend schmal sind, dass die Lagernadeln 120 nicht in Richtung auf die Längsachse 74 aus den Nadelaufnahmen 118c herausfallen können, sondern in diesen sicher gehalten werden.

Zum unverlierbaren Sichern der Rückhalteelemente 164 am Lagerkäfig 110c ist eine insgesamt mit dem Bezugszeichen 180 bezeichnete Kupplungseinrichtung 180 vorgesehen, welche erste Kupplungselemente 176 am Rückhalteelement 164 und zweite Kupplungselemente 178 am Lagerkäfig 110c umfasst. Die zweiten Kupplungselemente 178 sind in Form von schmalen Ausnehmungen in den endständigen Ringen 148c des Lagerkäfigs 110c ausgebildet. An jedem Ring 148c sind zwei zweite Kupplungselemente 178 vorgesehen, welche in Umfangsrichtung um 180° relativ zueinander versetzt ausgebildet sind.

Die ersten Kupplungselemente 176 sind in Form von in axialer Richtung voneinander weg weisenden Kupplungslappen 182 ausgebildet, welche von einer Ausgangsstellung, in welcher sie im Wesentlichen parallel zur Längsachse 74 weisen, in eine Kupplungsstellung bringbar sind, in welcher sie von freien Enden des Mittelstreifens 166 in radialer Richtung von der Längsachse 74 weg weisend abstehen. Von der Ausgangsstellung können sie in die Kupplungsstellung durch einfaches Umbiegen überführt werden. Eine Breite der Kupplungslappen 182 entspricht in etwa einer Breite der die zweiten Kupplungselemente 178 bildenden Aussparungen.

Zur Montage des Nadellagers 84c im Haltering 106 wird zunächst der Lagerkäfig 110c mit den beiden Kugeln 102 sowie den sechs Lagernadeln 120 bestückt und in den Haltering eingesetzt. Um die Kugeln 102 und die Lagernadeln 120 gegen Herausfallen zu sichern, werden die mit unverformten Kupplungslappen 182 hergestellten Rückhalteelemente 164 von entgegengesetzten Seiten und um 180° zueinander versetzt in den Lagerkäfig 110 eingeführt. Sobald die Rückhalteelemente 164 ihre Endposition einnehmen, werden die Kupplungslappen 182 umgebogen, so dass sie jeweils in die zweiten Kupplungselemente 178 eingreifen und so die Rückhalteelemente 164 axial am Lagerkäfig 110c sichern.

Die Lagerkäfige 110, 110a, 110b und 110c können wahlweise aus einem Metall oder aus einem Kunststoff hergestellt sein. Die Lagernadeln 120 sowie die Kugeln 102 sind wahlweise aus einem Metall, vorzugsweise aus einem Hartmetall, oder einer Keramik hergestellt. Aus Metall hergestellte Lagernadeln 120 beziehungsweise Kugeln 102 sind vorzugsweise mit einer Hartstoffbeschichtung versehen. Dabei kann es sich um ein Metall-Nitrid, insbesondere Titannitrid oder Chromnitrid enthaltende oder aus den genannten Materialien bestehende Beschichtung handeln.

## Patentansprüche

1. Chirurgisches Instrument (15) umfassend einen Schaft (62) und eine im Schaft (62) rotierbar gelagerte Antriebswelle (64), welche an ihrem distalen Ende ein Werkzeugelement (76) trägt oder umfasst, wobei im distalen Endbereich des Schafts (62) ein Radiallager (82) angeordnet oder ausgebildet ist zum rotierbaren Lagern der Antriebswelle (64) am Schaft (62), wobei das Radiallager (82) in Form eines Nadellagers (84) ausgebildet ist, wobei das Nadellager (84, 84a, 84b, 84c) einen Lagerkäfig (110, 110a, 110b, 110c) umfasst, in welchem eine Mehrzahl von Lagernadeln (120) parallel zu einer Längsachse (74) des Nadellagers (84, 84a, 84b, 84c) rotierbar gelagert sind, **dadurch gekennzeichnet, dass** der Lagerkäfig (110b) zwei miteinander verbundene Lagerkäfigteile (146) umfasst, dass das Instrument eine Verbindungseinrichtung (156) zum Verbinden der beiden Lagerkäfigteile (146) umfasst und dass das Instrument eine Nadellagersicherungseinrichtung (92) zum axialen Sichern des Nadellagers (84, 84a, 84b, 84c) am Schaft (62) umfasst.

2. Chirurgisches Instrument nach Anspruch 1, **dadurch gekennzeichnet, dass** die Nadellagersicherungseinrichtung (92) mindestens ein erstes Sicherungselement (94) und mindestens ein zweites Sicherungselement (96) umfasst, dass das eine der mindestens einen ersten und zweiten Sicherungselemente (94, 96) am Nadellager (84, 84a, 84b, 84c) angeordnet oder gehalten ist, dass das andere der mindestens einen ersten und zweiten Sicherungselemente (94, 96) am Schaft (62) angeordnet oder gehalten ist und dass das mindestes eine erste Sicherungselement (94) und das mindestens eine zweite Sicherungselement (96) in einer Sicherungsstellung miteinander in Eingriff stehen.

3. Chirurgisches Instrument nach Anspruch 1 oder 2, **dadurch gekennzeichnet, dass** das Nadellager (84, 84a, 84b, 84c) axial gesichert am Schaft (62) gehalten ist.

4. Chirurgisches Instrument nach einem der voranstehenden Ansprüche,
**dadurch gekennzeichnet, dass** der Lagerkäfig (110, 110a) aus einem mindestens teilweise elastischen Material hergestellt ist.

5. Chirurgisches Instrument nach einem der voranstehenden Ansprüche,
**dadurch gekennzeichnet, dass** der Lagerkäfig (110a, 110b) für jedes erste Sicherungselement (94) eine Sicherungselementaufnahme (112a) umfasst, in welcher das Sicherungselement (94) mit einem Halteglied (134a, 134b) gehalten ist.

6. Chirurgisches Instrument nach einem der voranstehenden Ansprüche,
**dadurch gekennzeichnet, dass** der Lagerkäfig (110b) aus zwei identischen Lagerkäfigteilen (146) ausgebildet ist.

7. Chirurgisches Instrument nach einem der voranstehenden Ansprüche,
**dadurch gekennzeichnet, dass** die Verbindungseinrichtung (156) in Form einer Rastverbindungseinrichtung (162) ausgebildet ist.

8. Chirurgisches Instrument nach einem der voranstehenden Ansprüche,
**dadurch gekennzeichnet, dass** die Verbindungseinrichtung (156) erste und zweite Verbindungselemente (152, 154) umfasst, welche jeweils an einem der beiden Lagerkäfigteile (146) angeordnet oder ausgebildet sind, welche ersten und zweiten Verbindungselemente (152, 154) in einer Trennstellung außer Eingriff stehen und in einer Verbindungsstellung miteinander in Eingriff stehen.

9. Chirurgisches Instrument nach Anspruch 8, **dadurch gekennzeichnet, dass** jeweils ein erstes und ein zweites Verbindungselement (152, 154) eine Sicherungselementaufnahme (112) mindestens teilweise begrenzen.

10. Chirurgisches Instrument nach einem der Ansprüche 2 bis 9 **dadurch gekennzeichnet, dass** das Nadellager (84c) mindestens ein Rückhalteelement (164) umfasst zum Sichern der Lagernadeln (120) am Lagerkäfig (110c).

11. Chirurgisches Instrument nach einem der voranstehenden Ansprüche, **gekennzeichnet durch** eine Kupplungseinrichtung (180) zum Kuppeln des mindestens einen Rückhalteelements (164) und des Lagerkäfigs (110c).

12. Chirurgisches Instrument nach einem der voranstehenden Ansprüche, **dadurch gekennzeichnet, dass** der Lagerkäfig (110, 110a, 110b, 110c) aus einem Metall oder aus einem Kunststoff hergestellt ist und/oder dass die Lagernadeln (120) und/oder die Sicherungselemente (94, 96) aus einem Metall hergestellt sind und/oder dass die Lagernadeln (120) und/oder die Sicherungselemente (94, 96) aus einer Keramik hergestellt sind und/oder dass die Lagernadeln (120) oder die Sicherungselemente (94, 96) mit einer Hartstoffbeschichtung versehen sind.

13. Chirurgisches Instrument nach Anspruch 12, **dadurch gekennzeichnet, dass** die Hartstoffbeschichtung ein Metall-Nitrid, insbesondere TiN oder CrN, ist oder enthält.

14. Chirurgisches Handstück (14) umfassend einen in einem Gehäuse angeordneten Antrieb, **gekennzeichnet durch** ein chirurgisches Instrument (15) nach einem der voranstehenden Ansprüche.

15. Chirurgisches Antriebssystem (10) umfassend mindestens ein chirurgisches Handstück (14) mit einem in einem Gehäuse angeordneten Antrieb und eine Steuer- und/oder Regelungsvorrichtung (12) zum Steuern und/oder Regeln des Antriebs, **gekennzeichnet durch** mindestens ein chirurgisches Instrument (15) nach einem der Ansprüche 1 bis 13.

## Claims

1. Surgical instrument (15) comprising a shank (62) and a drive shaft (64), which is rotatably mounted in the shank (62) and bears or comprises a tool element (76) at its distal end, wherein in the distal end region of the shank (62) a radial bearing (82) is arranged or configured for the rotatable mounting of the drive shaft (64) on the shank (62), wherein the radial bearing (82) is configured in the form of a needle bearing (84), wherein the needle bearing (84, 84a, 84b, 84c) comprises a bearing cage (110, 110a, 110b, 110c), in which a plurality of bearing needles (120) are rotatably mounted parallel to a longitudinal axis (74) of the needle bearing (84, 84a, 84b, 84c), **characterized in that** the bearing cage (110b) comprises two interconnected bearing cage parts (146), that the instrument comprises a connection device (156) for connecting the two bearing cage parts (146) and that the instrument comprises a needle bearing locking arrangement (92) for axially securing the needle bearing (84, 84a, 84b, 84c) on the shank (62).

2. Surgical instrument according to claim 1, **characterized in that** the needle bearing locking arrangement (92) comprises at least one first locking element (94) and at least one second locking element (96), that one of the at least one first and second locking elements (94, 96) is arranged or held on the needle bearing (84, 84a, 84b, 84c), that the other of the at least one first and second locking elements (94, 96) is arranged or held on the shank (62), and that the at least one first locking element (94) and the at least one second locking element (96) are in engagement with one another in a locking position.

3. Surgical instrument according to claim 1 or 2, **characterized in that** the needle bearing (84, 84a, 84b, 84c) is held axially secured on the shank (62).

4. Surgical instrument according to one of the preceding claims, **characterized in that** the bearing cage (110, 110a) is made from an at least partially elastic material.

5. Surgical instrument according to one of the preceding claims, **characterized in that** for each first locking element (94) the bearing cage (110a, 110b) comprises a locking element seating (112a), in which the locking element (94) is held with a holding member (134a, 134b).

6. Surgical instrument according to one of the preceding claims, **characterized in that** the bearing cage (110b) is configured from two identical bearing cage parts (146).

7. Surgical instrument according to one of the preceding claims, **characterized in that** the connection device (156) is configured in the form of a snap-in locking arrangement (162).

8. Surgical instrument according to one of the preceding claims, **characterized in that** the connection device (156) comprises first and second connection elements (152, 154), which are respectively arranged or formed on one of the two bearing cage parts (146), which first and second connection elements (152, 154) are disengaged in a separation position and are in engagement with one another in a connection position.

9. Surgical instrument according to claim 8, **characterized in that** a first and a second connection element (152, 154) respectively delimit a locking element seating (112) at least partially.

10. Surgical instrument according to one of claims 2 to 9, **characterized in that** the needle bearing (84c) comprises at least one retaining element (164) for securing the bearing needles (120) on the bearing cage (110c).

11. Surgical instrument according to one of the preceding claims, **characterized by** a coupling device (180) for coupling the at least one retaining element (164) and the bearing cage (110c).

12. Surgical instrument according to one of the preceding claims, **characterized in that** the bearing cage (110, 110a, 110b, 110c) is made from a metal or a plastic and/or that the bearing needles (120) and/or the locking elements (94, 96) are made from a metal and/or that the bearing needles (120) and/or the locking elements (94, 96) are made from a ceramic and/or that the bearing needles (120) or the locking elements (94, 96) are provided with a hard material coating.

13. Surgical instrument according to claim 12, **characterized in that** the hard material coating is or contains a metal nitride, in particular TiN or CrN.

14. Surgical handpiece (14) comprising a drive arranged in a housing, **characterized by** a surgical instrument (15) according to one of the preceding claims.

15. Surgical drive system (10) comprising at least one surgical handpiece (14) with a drive arranged in a housing and an open-loop and/or closed-loop control device (12) for open-loop and/or closed-loop control of the drive, **characterized by** at least one surgical instrument (15) according to one of claims 1 to 13.

## Revendications

1. Instrument chirurgical (15) comprenant un corps allongé (62) et un arbre d'entraînement (64), qui est monté en rotation dans le corps allongé (62), et porte ou comporte à son extrémité distale un élément d'outil (76), un palier radial (82) étant agencé ou formé dans la zone d'extrémité distale du corps allongé (62) pour le montage en rotation de l'arbre d'entraînement (64) dans le corps allongé (62), le palier radial (82) étant réalisé sous la forme d'un roulement à aiguilles (84), et le roulement à aiguilles (84, 84a, 84b, 84c) comprenant une cage de roulement (110, 110a, 110b, 110c) dans laquelle sont montées de manière rotative, parallèlement à un axe longitudinal (74) du roulement à aiguilles (84, 84a, 84b, 84c), une pluralité d'aiguilles de roulement (120), **caractérisé en ce que** la cage de roulement (110b) comprend deux parties de cage de roulement (146) assemblées l'une à l'autre, **en ce que** l'instrument comprend un dispositif d'assemblage (156) pour assembler les deux parties de cage de roulement (146), et **en ce que** l'instrument comprend un système d'arrêt de roulement à aiguilles (92) pour arrêter axialement le roulement à aiguilles (84, 84a, 84b, 84c) sur le corps allongé (62).

2. Instrument chirurgical selon la revendication 1, **caractérisé en ce que** le système d'arrêt de roulement à aiguilles (92) comprend au moins un premier élément d'arrêt (94) et au moins un deuxième élément d'arrêt (96), **en ce que** l'un desdits au moins un premier et deuxième éléments d'arrêt (94, 96) est agencé ou maintenu sur le roulement à aiguilles (84, 84a, 84b, 84c), **en ce que** l'autre desdits au moins un premier et deuxième éléments d'arrêt (94, 96) est agencé ou maintenu sur le corps allongé (62), et **en ce que** ledit au moins un premier élément d'arrêt (94) et ledit au moins un deuxième élément d'arrêt (96) sont en prise réciproque dans une position d'arrêt.

3. Instrument chirurgical selon la revendication 1 ou la revendication 2, **caractérisé en ce que** le roulement à aiguilles (84, 84a, 84b, 84c) est maintenu sur le corps allongé (62) en y étant arrêté axialement.

4. Instrument chirurgical selon l'une des revendications précédentes, **caractérisé en ce que** la cage de roulement (110, 110a) est fabriquée en un matériau au moins partiellement élastique.

5. Instrument chirurgical selon l'une des revendications précédentes, **caractérisé en ce que** la cage de roulement (110a, 110b) comprend pour chaque premier élément d'arrêt (94), un logement de réception d'élément d'arrêt (112a), dans lequel l'élément d'arrêt (94) est maintenu à l'aide d'un organe de maintien (134a, 134b).

6. Instrument chirurgical selon l'une des revendications précédentes, **caractérisé en ce que** la cage de roulement (110b) est réalisée en deux parties de cage de roulement (146) identiques.

7. Instrument chirurgical selon l'une des revendications précédentes, **caractérisé en ce que** le dispositif d'assemblage (156) est réalisé sous la forme d'un dispositif d'assemblage par encliquetage (162).

8. Instrument chirurgical selon l'une des revendications précédentes, **caractérisé en ce que** le dispositif d'assemblage (156) comprend des premiers et deuxièmes éléments d'assemblage (152, 154), qui sont agencés ou réalisés respectivement sur l'une des deux parties de cage de roulement (146), ces premiers et deuxièmes éléments d'assemblage (152, 154) étant hors de prise réciproque dans une position de séparation, et étant en prise réciproque dans une position d'assemblage.

9. Instrument chirurgical selon la revendication 8, **caractérisé en ce que** respectivement un premier et un deuxième élément d'assemblage (152, 154) délimitent au moins partiellement un logement de réception d'élément d'arrêt (112).

10. Instrument chirurgical selon l'une des revendications 2 à 9, **caractérisé en ce que** le roulement à aiguilles (84c) comprend au moins un élément de retenue (164) pour retenir les aiguilles de roulement (120) dans la cage de roulement (110c).

11. Instrument chirurgical selon l'une des revendications précédentes, **caractérisé par** un dispositif de couplage (180) pour assurer le couplage dudit au moins un élément de retenue (164) et de la cage de roulement (110c).

12. Instrument chirurgical selon l'une des revendications précédentes, **caractérisé en ce que** la cage de roulement (110, 110a, 110b, 110c) est fabriquée en un métal ou en une matière plastique, et/ou **en ce que** les aiguilles de roulement (120) et/ou les éléments d'arrêt (94, 96) sont fabriqués en un métal, et/ou **en ce que** les aiguilles de roulement (120) et/ou les éléments d'arrêt (94, 96) sont fabriqués en une céramique, et/ou **en ce que** les aiguilles de roulement (120) ou les éléments d'arrêt (94, 96) sont munis d'un revêtement de matériau dur.

13. Instrument chirurgical selon la revendication 12, **caractérisé en ce que** le revêtement de matériau dur est ou renferme un nitrure métallique, notamment TiN ou CrN.

14. Poignée chirurgicale (14) comprenant un entraînement agencé dans un boitier, **caractérisée par** un instrument chirurgical (15) selon l'une des revendications précédentes.

15. Système d'entraînement chirurgical (10) comprenant au moins une poignée chirurgicale (14) avec un entraînement agencé dans un boitier, et un dispositif de commande et/ou de régulation (12) pour commander et/ou réguler l'entraînement, **caractérisé par** au moins un instrument chirurgical (15) selon l'une des revendications 1 à 13.
